# EUROPEAN PATENT APPLICATION

(11) **EP 1 787 975 A1**
(43) Date of publication of application: **23.05.2007**
(21) Application number: 04773250.8
(22) Date of filing: 10.09.2004
(51) Int. Cl.: C07C 67/31, C07C 69/732, C07D 307/83, A23L 1/03, A23L 1/226, C11B 9/00

(54) **PROCESSES FOR PRODUCTION OF WINE LACTONE AND ITS INTERMEDIATES AND APPLICATION OF THE LACTONE**

(71) Applicant: SAN-EI GEN F.F.I., INC., Toyonaka-shi, Osaka 561-8588 (JP)
(72) Inventor: OMOTO, Mineko, SAN-EI GEN F.F.I., INC., Toyonaka-shi, Osaka 561-8588 (JP); YUKAWA, Chiyoki, SAN-EI GEN F.F.I., INC., Toyonaka-shi, Osaka 561-8588 (JP); NISHIJO, Yoshiteru, SAN-EI GEN F.F.I., INC., Toyonaka-shi, Osaka 561-8588 (JP)
(74) Representative: Müller-Boré & Partner Patentanwälte
(86) International application number: PCT/JP2004/013606
(87) International publication number: WO 2006/027856

(57) **Abstract**

The present invention provides a method for producing wine lactone, a method for producing intermediates that are usable in production of wine lactone, and techniques for the application of wine lactone. The method for producing the wine lactone (Compound (6)) of the present invention comprises the following steps (A) to (E): 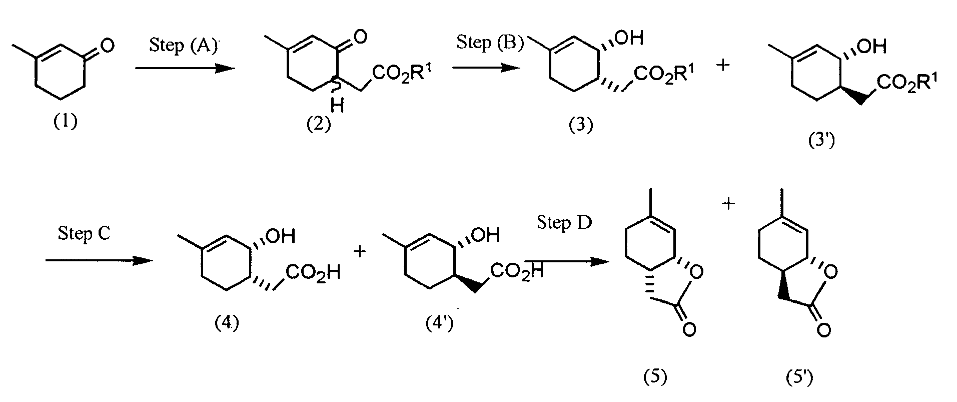 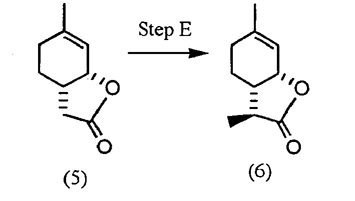 wherein R¹ is a C₁₋₄ lower alkyl group.

## Description

### TECHNICAL FIELD

The present invention relates to a method for producing a wine lactone, a method for producing an intermediate usable for producing the wine lactone, and the application of the wine lactone.

### BACKGROUND OF THE INVENTION

Wine lactone [(3S,3aS,7aR)-3,6-dimethyl-3a,4,5,7a-tetrahydro-1-benzofuran-2(3H)-one], which is a compound exists in nature, was isolated in 1975 by I. A. Southwell as a metabolite of koalas, and named "wine lactone" because it revealed that this compound was also contained in white wine in 1996. Wine lactone has eight stereoisomers. All eight stereoisomers were synthesized by H. Guth in 1996, and it was confirmed by chiral analysis that the compound that exists in nature is (3S,3aS,7aR) form, i.e., [(3S,3aS,7aR)-3,6-dimethyl-3a,4,5,7a-tetrahydro-1-benzofuran-2(3H)-one: Compound (6)], represented by the formula below (Helv.Chim.Acta, 79, 1559, (1996)) .

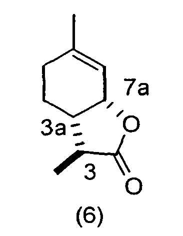

Examples of known methods for producing wine lactone include a method proposed by H. Guth wherein all stereoisomers are synthesized; a method utilizing 6-membered ring formation reaction via the Diels-Alder reaction; and a method wherein limonene, which has the same stereochemistry as that of the methyl group at the 3a-posiotion of wine lactone, is used as a starting material (Helv. Chim. Acta, 79, 1559 (1996)). Among these, in the method using the Diels-Alder reaction, isolation of end product is extremely difficult because of the low yield of the oxidation reaction. Furthermore, in the method using limonene having the same stereochemistry as that of the 3a-posiotion of wine lactone as a starting material, a mixture of stereoisomers at the 3-posiotion is formed and isolation thereof is difficult.

The method for synthesizing wine lactone proposed by P. A. Bartlett, C. F. Pizzo, et al. (J. Org. Chem., 46, 3869-3900 (1981)), which is similar to that described immediately above, is good in that the stereochemistry at the 3a-position can be completely controlled by using the rearrangement reaction from an optically active alcohol, and therefore the targeted stereoisomer, among several stereoisomers, can be selectively synthesized; however, it is extremely difficult to obtain the starting material, i.e., an optically active alcohol, and therefore this method cannot be employed for industrial mass-production.

E. J. Bergner, G. Helmchen et al. (Eur. J. Org. Chem., 419-423 (2000)) proposed a method wherein only the naturally occurring-type wine lactone is obtained by conducting an addition reaction of malonic ester with palladium utilizing an optically active ligand. This method comprises several steps such as a lactonization step, lactone-cleaving step, and a recyclization step. So many steps result in a low yield of the targeted product.

The method proposed by S. P. Chavan et al. (Tetrahedron Asymmetry, 12, 2985-2988 (2001)) is to synthesize the wine lactone using the conversion of limonene having the same stereochemistry as that of the 3a-position of naturally occurring-type wine lactone. This method has a problematic low yield because a stereoisomeric mixture of the methyl group at the 3-position is obtained and stereoisomers are formed as byproducts.

### DISCLOSURE OF THE INVENTION

In view of the problems of the prior art techniques described above, the present inventors conducted extensive research to provide a method that can solve these problems and selectively produce the naturally occurring type of wine lactone (compound (6)). The present inventors found that, [(3S,3aS,7aR)-3,6-dimethyl-3a,4,5,7a-tetrahydro-1-benzofuran-2(3H)-one: Compound (6)], which is the naturally occurring type of wine lactone, can be selectively produced at a high yield out of eight types of wine lactone stereoisomers, by employing in the wine lactone production process a step of reacting 6-(carboalkoxymethyl)-3-methyl-2-cyclohexene-1-one (Compound (2)) with an optically active oxazaborolidine, which is used as an asymmetric catalyst, and reducing the carbonyl group of the compound to obtain an alcohol, i.e., [(1R,6S)-6-(carboalkoxymethyl)-3-methyl-2-cyclohexenel-ol] (Compound (3)).

The present inventors also found that the wine lactone can be used in flavors, perfumes and food products, and thus completed the present invention.

The present invention provides the following:
Item 1. A method for producing (1R,6S)-6-(carboalkoxymethyl)-3-methyl-2-cyclohexene-1-ol (Compound (3)) represented by the formula below: 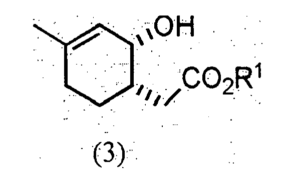 wherein R¹ is a C₁₋₄ lower alkyl group;
   the method comprising the step of reducing the carbonyl group in 6-(carboalkoxymethyl)-3-methyl-2-cyclohexene-1-one (Compound (2)) represented by the formula below: 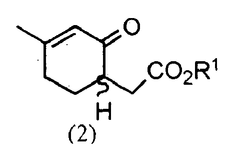 wherein R¹ is the same as the above;
   by using an optically active oxazaborolidine as an asymmetric catalyst.
Item 2. The method for producing (1R,6S)-6-(carboalkoxymethyl)-3-methyl-2-cyclohexene-1-ol (Compound (3)) according to Item 1, wherein the optically active oxazaborolidine is (R)-5,5-diphenyl-2-methyl-3,4-propano-1,3,2-oxazaborolidine.
Item 3. A method for producing (3S,3aS,7aR)-3,6-dimethyl-3a,4,5,7a-tetrahydro-1-benzofuran-2(3H)-one (Compound (6)) comprising steps (A) to (E) below:
   (1) Step (A): subjecting 3-methyl-2-cyclohexene-1-one (Compound (1)) to an alkyl addition reaction to obtain 6-(carboalkoxymethyl)-3-methyl-2-cyclohexene-1-one (Compound (2));
   (2) Step (B): reducing the carbonyl group in the thus-obtained 6-(carboalkoxymethyl)-3-methyl-2-cyclohexene-1-one (Compound (2)) using an optically active oxazaborolidine as an asymmetric catalyst to obtain (1R,6S)-6-(carboalkoxymethyl)-3-methyl-2-cyclohexene-1-ol (Compound (3));
   (3) Step (C): hydrolyzing the ester moiety of the thus-obtained (1R,6S)-6-(carboalkoxymethyl)-3-methyl-2-cyclohexene-1-ol (Compound (3)) to obtain (1R,6S)-6-carboxymethyl -3-methyl-2-cyclohexene-1-ol (Compound (4));
   (4) Step (D): lactonizing (1R,6S)-6-carboxymethyl-3-methyl-2-cyclohexene-1-ol (Compound (4)) to obtain (3aS,7aR)-tetrahydro-6-methyl-2(3H)-benzofuranone (Compound (5)); and
   (5) Step (E): methylasing (3aS,7aR)-tetrahydro-6-methyl-2(3H)-benzofuranone (Compound (5)) to obtain (3S,3aS,7aR)-3,6-dimethyl-3a,4,5,7a-tetrahydro-1-benzofuran-2(3H)-one (Compound (6)). 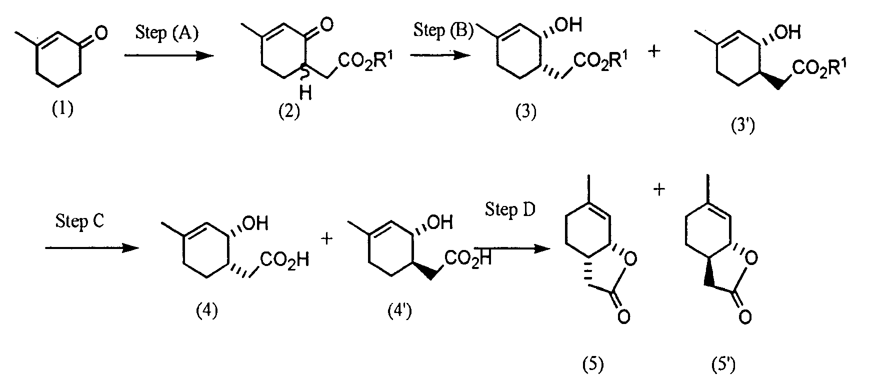 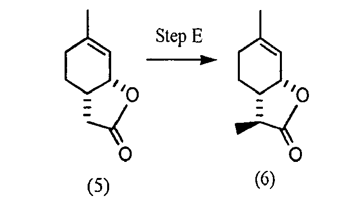
   wherein R¹ is a C₁₋₄ lower alkyl group.
Item 4. The method for producing (3S,3aS,7aR)-3,6-dimethyl-3a,4,5,7a-tetrahydro-1-benzofuran-2(3H)-one (Compound (6)) according to Item 3, wherein the optically active oxazaborolidine used in Step (B) is (R)-5,5-diphenyl-2-methyl-3,4-propano-1,3,2-oxazaborolidine.
Item 5. A (3S,3aS,7aR)-3,6-dimethyl-3a,4,5,7a-tetrahydro-1-benzofuran-2(3H)-one obtained by the method of Item 4.
Item 6. A flavoring or perfume composition containing a flavoring or perfume component and 10⁻⁷ to 10⁴ ppb of (3S,3aS,7aR)-3,6-dimethyl-3a,4,5,7a-tetrahydro-1-benzofuran-2(3H)-one.
Item 7. The flavoring or perfume composition according to Item 6, wherein the flavoring or perfume component is a fruit-based flavoring or perfume component.
Item 8. A method for improving the texture of a flavoring or perfume composition comprising the step of adding (3S,3aS,7aR)-3,6-dimethyl-3a,4,5,7a-tetrahydro-1-benzofuran-2(3H)-one to the flavoring or perfume composition in addition to a flavoring or perfume component in such a manner that the concentration of the (3S,3aS,7aR)-3,6-dimethyl-3a,4,5,7a-tetrahydro-1-benzofuran-2(3H)-one is 10⁻⁷ to 10⁴ ppb.
Item 9. The method according to Item 8, wherein the texture is a flavor or fragrance, heavy-texture, or juicy-taste.
Item 10. Foods and beverages containing 10⁻¹⁰ to 10² ppb of (3S,3aS,7aR)-3,6-dimethyl-3a,4,5,7a-tetrahydro-1-benzofuran-2(3H)-one.
Item 11. A method for improving the flavor of foods and beverages comprising the step of adding 10⁻¹⁰ to 10² ppb of (3S,3aS,7aR)-3,6-dimethyl-3a,4,5,7a-tetrahydro-1-benzofuran-2(3H)-one to the foods and beverages.
Item 12. The method for improving the flavor of foods and beverages according to Item 11, wherein the flavor is a juicy-taste or a heavy-texture.

### BEST MODE FOR CARRYING THE INVENTION

### (1) Preparation of 6-(carboalkoxymethyl)-3-methyl-2-cyclohexene-1-ol

The present invention provides a method of preparing 6-(carboalkoxymethyl)-3-methyl-2-cyclohexene-1-ol, which is an intermediate product for production of wine lactone. Here, 6-(carboalkoxymethyl)-3-methyl-2-cyclohexene-1-ol includes (1R,6S)-6-(carboalkoxymethyl)-3-methyl-2-cyclohexene-1-ol (Compound (3)) and (1R,6R)-6-(carboalkoxymethyl)-3-methyl-2-cyclohexene-1-ol (Compound (3')), which is a stereoisomer of (1R,6S)-6-(carboalkoxymethyl)-3-methyl-2-cyclohexene-1-ol (Compound (3)).

As shown in the formula below, the method for producing 6-(carboalkoxymethyl)-3-methyl-2-cyclohexene-1-ol of the present invention is characterized in that the carbonyl group of 6-(carboalkoxymethyl)-3-methyl-2-cyclohexene-1-one (Compound (2)) is reduced by using an optically active oxazaborolidine as an asymmetric catalyst.

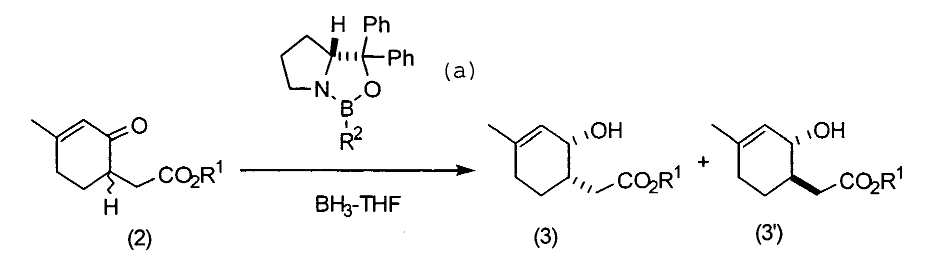

In the above formula, R¹ is a C₁₋₄ lower alkyl group, and R² is a hydrogen atom or a methyl group.

Examples of alkyl groups represented by R¹ in the 6-(carboalkoxymethyl)-3-methyl-2-cyclohexene-1-one (Compound (2)), which is used as a starting material, include methyl group, ethyl group, propyl group, isopropyl group, butyl group, isobutyl group and like straight or branched chain C₁₋₄ lower alkyl groups. Among these, the methyl, ethyl, and propyl groups are preferable.

There is no limitation on Compound (2), and it can be prepared by, for example, reacting 3-methyl-2-cyclohexene-1-one (Compound (1)) with lithium diisopropylamide (LDA) and halogenated alkyl acetate as described later.

The optically active oxazaborolidine used as an asymmetric catalyst in the reaction of Compound (2) is a compound having the structure shown as Formula (a) below.

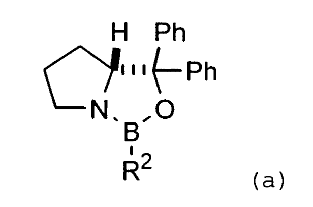

R² in Formula (a) may be a hydrogen atom or a methyl group. (R)-5,5-diphenyl-2-methyl-3,4-propano-1,3,2-oxazaborolidine having a methyl group is preferable.

By using such optically active oxazaborolidine as an asymmetric catalyst, it is possible to stereoselectively control the hydroxyl group at the 1-position of [6-(carboalkoxymethyl)-3-methyl-2-cyclohexene-1-ol] (alcohol) that is produced by reducing the carbonyl group of Compound (2) in such a manner that R-isomers can be obtained (i.e., control of stereostructure).

In the production of wine lactone described later, among [6-(carboalkoxymethyl)-3-methyl-2-cyclohexene-1-ol] alcohols mentioned above, [(1R,6S)-6-(carboalkoxymethyl)-3-methyl-2-cyclohexene-1-ol] (Compound (3)), which is a (1R,6S) isomer, can be effectively used as an intermediate. In this case, it is preferable to use (R)-5,5-diphenyl-2-methyl-3,4-propano-1,3,2-oxazaborolidine as an optically active oxazaborolidine.

Such a reduction reaction is preferably conducted under an inert atmosphere such as nitrogen gas, argon gas, etc., and particularly preferably under an argon gas atmosphere. There are no particular limitations to the reaction temperature and the reaction time, but the reaction is generally conducted in such a manner that the above-mentioned asymmetric catalyst (optically active oxazaborolidine) and a solvent are placed in a reaction vessel, Compound (2) dissolved in the same solvent is added to the mixture in the reaction vessel, cooled to about -30 to 30°C, and then stirred in the presence of a reducing agent for about 5 to 60 minutes. There is no particular limitation to the amount of asymmetric catalyst (optically active oxazaborolidine) used, but the amount is generally 0.05 to 0.5 mol, and preferably 0.05 to 0.1 mol per 1 mol of Compound (2), which is a starting material. Note that such an asymmetric catalyst can be recovered by a known method and reused.

There is no limitation to the reducing agent used, but a borane-tetrahydrofuran complex is preferably used. There is no particular limitation to the solvent used, but preferable examples thereof include tetrahydrofuran, diethyl ether and like ether-type solvents.

### (2) Method for Producing Wine Lactone (Compound (6))

The present invention also provides a method for producing wine lactone (Compound (6)). The method for producing wine lactone (Compound (6)) of the present invention comprises Steps (A) to (E) shown in the formulae below: 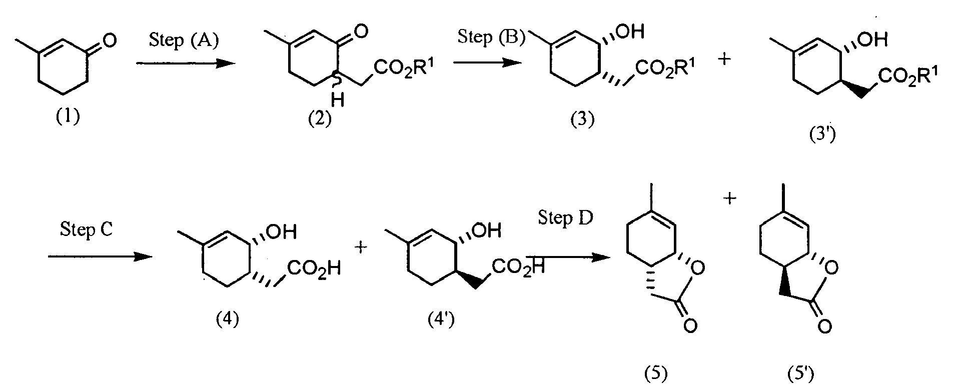 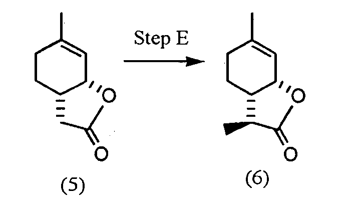 wherein R¹ is a C₁₋₄ lower alkyl group.

Note that such production method comprises, as Step (B), the method for producing 6-(carboalkoxymethyl)-3-methyl-2-cyclohexene-1-ol (Compounds (3), (3')) from 6-(carboalkoxymethyl)-3-methyl-2-cyclohexene-1-one (Compound (2)) of the present invention described above.
(i) Step (A): A step of producing 6-(carboalkoxymethyl)-3-methyl-2-cyclohexene-1-one (Compound (2)) from 3-methyl-2-cyclohexene-1-one (Compound (1)).
   Step (A) is a reaction to obtain an alkyl adduct (i.e., Compound (2)) by subjecting 3-methyl-2-cyclohexene-1-one (Compound (1)) to an alkyl addition reaction by reacting with lithium diisopropylamide (LDA) and halogenated alkyl acetate.
   There is no limitation to the usable halogenated alkyl acetate, but specific examples thereof include methyl bromoacetate, ethyl bromoacetate, propyl bromoacetate, isopropyl bromoacetate, butyl bromoacetate, isobutyl bromoacetate, methyl chloroacetate, ethyl chloroacetate, propyl chloroacetate, isopropyl chloroacetate, butyl chloroacetate, isobutyl chloroacetate, etc.
   This step can be specifically conducted by using 1 to 2 mol of lithium diisopropylamide and 1 to 2 mol of halogenated alkyl acetate per 1 mol of Compound (1), and reacting them at - 78°C to +30°C for 12 to 36 hours. Said reaction is conducted in a solvent, preferably a diethyl ether, tetrahydrofuran or the like ether-type solvent. In this case, it is preferable that hexamethylphosphoramide (HMPA) be added to the solvent.
(ii) Step (B): A step of producing 6-(carboalkoxymethyl)-3-methyl-2-cyclohexene-1-ol (Compounds (3) and (3')) from 6-(carboalkoxymethyl)-3-methyl-2-cyclohexene-1-one (Compound (2)).
   Step (B) can be conducted by the method described in Item (1) above. Specifically, Step (B) can be conducted by reducing the carbonyl group of 6-(carboalkoxymethyl)-3-methyl-2-cyclohexene-1-one (Compound (2)) using an optically active oxazaborolidine (Compound (a)) as an asymmetric catalyst. A preferable example of optically active oxazaborolidine is (R)-5,5-diphenyl-2-methyl-3,4-propano-1,3,2-oxazaborolidine. By using such optically active oxazaborolidine, (1R,6S)-6-(carboalkoxymethyl)-3-methyl-2-cyclohexene-1-ol (Compound (3)) can be produced as a main product. Note that, in this case, (1R,6R)-6-(carboalkoxymethyl)-3-methyl-2-cyclohexene-1-ol (Compound (3')), which is a byproduct produced in Step (B), is a stereoisomer of Compound (3).
(iii) Step (C): A step of producing a hydroxy acid [Compound (4): (1R,6S)-6-carboxymethyl-3-methyl-2-cyclohexene-1-ol] from 6-(carboalkoxymethyl)-3-methyl-2-cyclohexene-1-ol (Compound (3)).
   Step (C) is a step wherein a hydroxy acid [Compound (4): (1R,6S)-6-carboxymethyl-3-methyl-2-cyclohexene-1-ol] is produced by hydrolyzing the ester moiety of 6-(carboalkoxymethyl)-3-methyl-2-cyclohexene-1-ol (Compound (3)) obtained in Step (B).
   Step (C) can specifically be conducted by dissolving Compound (3) in 10 to 30 w/v% aqueous sodium hydroxide or potassium hydroxide solution, reacting them at 0 to 30°C for 0.5 to 1 hour, and then neutralizing the resultant mixture using 10 to 30 w/v% aqueous hydrochloric acid solution. The amount of sodium chloride or potassium chloride used is preferably 1 to 2 mol per 1 mol of Compound (3). In this step, Compound (3'), i.e., ((1R,6R)-6-(carboalkoxymethyl)-3-methyl-2-cyclohexene-1-ol), which is a byproduct of Step (B), is also hydrolyzed, and Compound (4'), i.e., ((1R,6R)-6-carboxymethyl-3-methyl-2-cyclohexene-1-ol) is produced as a byproduct.
(iv) Step (D): A step of producing lactone (Compound (5)) from hydroxy acid (Compound (4)).
   Step (D) is conducted to lactonize the hydroxy acid (Compound (4), i.e., (1R,6S)-6-carboxymethyl-3-methyl-2-cyclohexene-1-ol) obtained in Step (C) by reacting with dicyclohexylcarbodiimide (DCC). A lactone (Compound (5): (3aS,7aR)-tetrahydro-6-methyl-2(3H)-benzofuranone) can be thus obtained.
   Step (D) can specifically be conducted by dissolving Compound (4) in benzene, adding dicyclohexylcarbodiimide (DCC), and being allowed to react at 0 to 30°C for about 10 to 30 hours. The amount of DCC used is preferably 1 to 2 mol per 1 mol of Compound (4).
   In this case, Compound (4'), i.e., ((1R,6R)-6-carboxymethyl-3-methyl-2-cyclohexene-1-ol), which is a byproduct produced by Step (C), is also lactonized, and Compound (5')[(3aR,7aR)-tetrahydro-6-methyl-2(3H)-benzofuranone] is produced as a byproduct. However, separation between the main product (Compound (5)) and the byproduct (Compound (5')) can be easily conducted. There is no limitation to the separation method, but a standard method such as column chromatography can be employed. Specific examples of column chromatography include silica gel column chromatography using hexane and ethyl acetate as developing solvents.
(v) Step (E): A step of producing wine lactone (Compound (6)) from lactone (Compound (5)).

Step (E) can be conducted by methylating the lactone (Compound (5)) obtained in Step (D) using methyl halide. Wine lactone [Compound (6): (3S,3aS,7aR)-3,6-dimethyl-3a,4,5,7a-tetrahydro-1-benzofuran-2(3H)-one] can be thus obtained.

Step (E) can specifically be conducted by using 1 to 2 mol of lithium diisopropylamide (LDA) and 1 to 2 mol of methyl halide per 1 mol of Compound (5), and being allowed to react at - 78°C to + 30°C for about 0.5 to 1 hour. Note that there is no limitation to the methyl halide used, but methyl bromide and methyl iodide are preferable.

All these steps (Steps (A) to (E)) except for Step (B) are conducted by known reactions; however, according to the present invention, by conducting the reactions of steps (A) to (E) by combing these steps, it is possible to selectively produce and obtain naturally occurring-type wine lactone ((3S,3aS,7aR)-3,6-dimethyl-3a,4,5,7a-tetrahydro-1-benzofuran-2(3H)-one: Compound (6)) regardless of the fact that a plurality of optical isomers exist. As a result, very pure wine lactone can be produced at a high yield.

### (3) Application of Wine Lactone

The present invention also provides a flavoring or perfume composition containing wine lactone. There is no limitation to the objects to which the flavoring or perfume composition of the present invention can be added, and specific examples thereof include not only cosmetics and toiletries such as soaps, perfumes, fragrances, shampoos, etc., but also foods and beverages, oral compositions, etc., which are taken or used orally. Flavoring compositions for use in foods and beverages are preferable.

Many flavors or perfumes, not only synthetic flavors or perfumes, but also natural flavors or perfumes have problems relating to a lack of satisfactory heavy-texture, and in the case of fruit-based flavors, loss of juicy-taste. The present invention has been accomplished based on the findings that, by adding wine lactone to flavors or perfumes, these problems can be solved, and not only heavy-texture and juicy-taste can be improved but also the flavor or fragrance of the original material can be reproduced well, increasing reality.

A main feature of the flavoring or perfume composition of the present invention is that it contains wine lactone (Compound (6)) in addition to a flavoring or perfume component, and the content of the wine lactone (Compound (6)) is not limited as long as the above effects can be achieved. However, the content of the wine lactone in the flavoring or perfume composition is preferably about 10⁻⁷ to 10⁴ ppb, and more preferably about 10⁻⁶ to 10³ ppb.

Note that the flavoring or perfume composition of the present invention may comprise, in addition to the flavoring or perfume component and wine lactone, additives and solvents usually used in flavors and perfumes. Examples of usable solvents include ethanol, propylene glycol, etc. There is no limitation to the flavoring or perfume component used in the present invention, and all known flavoring or perfume components are included. Fruit-based flavors or perfumes are preferable, and citrus fruit-based flavors or perfumes (grapefruit, lemon, orange, etc.) are particularly preferable.

The present invention also provides foods and beverages containing wine lactone. There is no limitation to the foods and beverages, but examples thereof include beverages, ice creams, sherbets and like frozen desserts; jelly, pudding, mizuyokan (sweet jellied bean paste), kuzukiri (arrowroot noodle) and like desserts; cookies, cakes, chocolates, chewing gums, manju (buns with a bean-jam filling) and like confectionaries; kashipan (pastry with fillings and flavors), white bread and like breads; jam, flour paste and like fillings; ramune-candies, tablets and like tablet candies. Among these, beverages are preferable and fruit juice-containing beverages, fruit juice-containing carbonated beverages, etc. are particularly preferable.

By adding wine lactone (Compound (6)) to these foods and beverages, the flavors that such foods and beverages inherently possess are improved and, in particular for fruit juice-containing beverages, juicy-taste and heavy-texture can be attained.

A main feature of the foods and beverages provided by the present invention is that they contain wine lactone, and the content of the wine lactone (Compound (6)) is not limited as long as the above-mentioned effects can be achieved. However, the content of the wine lactone in foods and beverages is preferably about 10⁻¹⁰ to 10² ppb, and more preferably about 10⁻⁹ to 10 ppb.

Note that the wine lactone can be added, in addition to foods and beverages, to dentifrice, mouthwash, lip balm, lipstick and like oral compositions; soaps, perfumes, fragrances, shampoos and like toiletries and cosmetics to improve the fragrance.

### EXAMPLES

Hereunder, the present invention is explained in detail below with reference to Examples. However, the scope of the present invention is not limited to these Examples.

### Example 1: Production of Wine Lactone

Wine lactone ((3S,3aS,7aR)-3,6-dimethyl-3a,4,5,7a-tetrahydro-1-benzofuran-2(3H)-one: Compound (6)) was produced by conducting Steps (A) to (E) shown in the formulae below.

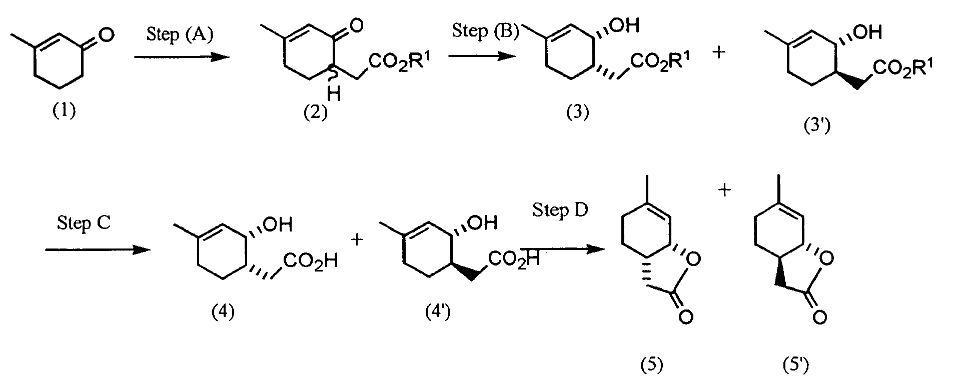

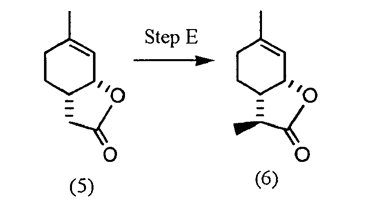

### (1) Step (A): Alkyl addition reaction

Diisopropylamine (16.8 ml, 0120 mmol) was dissolved in 400 ml of tetrahydrofuran, cooled to 0°C, and n-butyl lithium (1.56 mol/l, 76 ml) was added dropwise. To the resultant mixture, 3-methyl-2-cyclohexene-1-one (Compound (1), 11.3 ml, 100 mmol) was added dropwise, 50 ml of hexamethylphosphoramide was added, ethyl bromoacetate (16.6 ml, 150 mmol) was added dropwise, and then the mixture was reacted for 1.5 hours. The reaction was quenched using an aqueous saturated ammonium chloride solution, and extraction was conducted using ethyl acetate. The formed oil layer was washed with an aqueous saturated sodium chloride solution, dried using anhydrous sodium sulfate, and the solvent was removed under reduced pressure. The resultant residue was subjected to purification by distillation, and the fractions at specific boiling point, i.e., 101 to 105°C/0.1 mmHg, were collected. An alkyl group-added ketoester (Compound (2): 6-(carboethoxymethyl)-3-methyl-2-cyclohexene-1-one, 51.7 mmol, yield at 52%) was thereby obtained.
· Alkyl Group-added Ketoester (Compound (2)) [However, an alkyl group is an ethyl group]
   ¹H-NMR (CDCl₃) : d=1.27 (3H, t, J=7.1Hz), 1.79 (1H, qd, J=11.7, 4.9Hz), 1.96 (3H, s), 2.12 (1H, dtd, J=12.4, 4.9, 2.7Hz), 2.27 (1H, dd, J=16.1, 7.6Hz), 2.26-2.32 (1H, m), 2.41-2.50 (1H, m), 2.72-2.79 (1H, m), 2.89 (1H, dd, J=16.1, 5.4Hz), 4.16 (2H, qd, J=7.1, 2.0Hz), 5.88 (1H, s).
   ¹³C-NMR (CDCl₃) : d =14.11, 24.12, 28.40, 30.95, 34.49, 42.50, 60.37, 125.9, 162.0, 172.6, 199.1.

### (2) Step (B): Reduction of Carbonyl Group of Compound (2) Using Oxazaborolidine

Tetrahydrofuran (1 ml) and (R)-5,5-diphenyl-2-methyl-3,4-propano-1,3,2-oxazaborolidine (277 mg, 1 mmol) were placed in a reaction vessel under nitrogen atmosphere, and 2 ml of borane-tetrahydrofuran complex (1.13 mol/l) was added dropwise. To the thus-obtained mixture was added tetrahydrofuran solution (10.6 ml) of ketoester (Compound (2), 3.92 g, 20 mmol) prepared in Step (A). The resultant mixture was cooled to 0°C, and borane-tetrahydrofuran complex (8.6 ml) was added thereto dropwise. The reaction solution was stirred for 15 minutes, 3 ml of methanol was added thereto, 0.4 ml of diethyl ether saturated with sodium hydrogen was added dropwise, and then stirred for 30 minutes. The solvent was removed under reduced pressure, 10 ml of benzene was added and condensed under reduced pressure, this step being repeated twice. Diethyl ether (20 ml) was added, and cooled to 0°C. The deposited white crystals were filtered, and the filtrate was washed with a saturated aqueous sodium chloride solution, a saturated aqueous sodium carbonate solution, and a saturated aqueous sodium chloride solution, and dried using anhydrous sodium hydrogen sulfate. The solvent was removed under reduced pressure, and purification was conducted using silica gel column chromatography (with a developing solvent of hexane: ethyl acetate = 2 : 1 (v/v)), obtaining a mixture (12.5 mmol, yield at 63%) of Compound (3) [(1R,6S)-6-carboethoxymethyl-3-methyl-2-cyclohexene-1-ol] and Compound (3') [(1R,6R)-6-carboethoxymethyl-3-methyl-2-cyclohexene-1-ol].
· Compound (3)
   ¹³C-NMR (CDCl₃) : d =14.13, 23.01, 26.35, 29.25, 35.98, 37.92, 60.36, 71.37, 124.5, 137.2, 173.6.
· Compound (3')
   ¹³C-NMR (CDCl₃) : d =14.13, 23.29, 23.33, 30.01, 36.32, 39.04, 60.26, 65.89, 123.0, 139.0, 173.6.

### (3) Step (C): Hydrolysis of Ester moieties of Compounds (3) and (3')

A mixture (250 mg, 1.26 mmol) of Compound (3) and Compound (3') prepared in Step (B) was dissolved in 5 ml of methanol, and then 2 ml of 30% aqueous sodium hydroxide solution was added. Ten minutes after, the mixture was made acidic using 30% hydrochloric acid, and extraction was conducted using diethyl ether. The formed oil layer was washed with a saturated sodium chloride solution, and then dried using anhydrous sodium sulfate. The solvent was removed under reduced pressure, obtaining a coarse product of a mixture of Compound (4) and Compound (4'), which are hydroxy acids.

### (4) Step (D): Lactonizing Reaction Using DCC

A mixture (about 1.26 mmol) of Compound (4) and Compound (4') prepared in Step (C) was dissolved in 5 ml of benzene, dicyclohexylcarbodiimide (DCC, 287 mg, 1.39 mmol) was added, and stirred at 20°C for 15 hours. After the completion of the reaction, n-hexane was added, the deposited solid was filtered, the filtrate was washed with 1 N-hydrochloric acid and a saturated aqueous sodium chloride solution, dried using anhydrous sodium sulfate, and then the solvent contained therein was removed under reduced pressure. The resultant mixture was purified using silica gel column chromatography (with a developing solvent of hexane : ethyl acetate = 3 : 1 (v/v)), lactone (Compound (5)) [(3aS,7aR)-tetrahydro-6-methyl-2(3H)-benzofuranone] (0.64 mmol, yield at 51%) and lactone (Compound (5'))[(3aR,7aR)-tetrahydro-6-methyl-2(3H)-benzofuranone](0.17 mmol, 13%) were thereby separately isolated.
· Lactone (Compound (5))
   ¹H-NMR (CDCl₃) : d =1.44-1.54 (1H, m), 1.72-1.76 (1H, m), 1.78 (3H, s), 2.02 (2H, t, J=4.9Hz), 2.29 (1H, dd, J=17.1, 3.4Hz), 2.46-2.54 (1H, m), 2.72 (1H, dd, J=17.1, 8.1Hz), 4.80 (1H, t, J=4.4Hz), 5.62 (1H, m).
   ¹³C-NMR (CDCl₃) : d =23.92, 24.13, 28.02, 33.09, 35.49, 76.94, 117.7, 143.1, 176.9.
· Lactone (Compound (5'))
   ¹H-NMR (CDCl₃) : d =1.60-1.69 (1H, m), 1.70 (3H, s), 2.03-2.08 (1H, m), 2.11-2.21 (2H+1H, m), 2.27 (1H, dd, J=13.4, 15.9Hz), 2.54 (1H, ddd, J=15.9, 6.1, 1.0Hz), 4.41-4.44 (1H, m), 5.82 (1H, m).
   ¹³C-NMR (CDCl₃) : d =22.90, 23.62, 30.80, 35.73, 42.12, 82.50, 120.0, 137.9, 176.9.

### (5) Step (E): Methylation Reaction of Lactone (Compound (5))

Diisopropylamine (0.1 ml, 0.71 mmol) was dissolved in 1 ml of tetrahydrofuran, cooled to 0°C, and 0.46 ml of n-butyl lithium (1.56 mol/l) was added dropwise. To the thus-obtained mixture, a solution obtained by dissolving the lactone (Compound (5), 90 mg, 0.59 mmol) prepared in Step (D) in 1 ml of tetrahydrofuran was added dropwise, and then methyl iodide (0.05 ml, 0.71 mmol) was also added dropwise. After 20 minutes from the initiation of the reaction, the reaction was quenched using water, and extraction was conducted using diethyl ether. The formed oil layer was washed with a saturated aqueous sodium chloride solution, dried using anhydrous sodium sulfate, and the solvent was removed under reduced pressure. Purification was conducted using silica gel column chromatography (with a developing solvent of hexane: ethyl acetate = 4 : 1 (v/v)), obtaining naturally occurring-type wine lactone (Compound (6): (3S,3aS,7aR)-3,6-dimethyl-3a,4,5,7a-tetrahydro-1-benzofuran-2(3H)-one.
· Wine Lactone (Compound (6))
   ¹H-NMR (C₆D₆) : d =0.98 (3H, d, J=7.3Hz), 1.16-1.21 (2H, m), 1.41 (3H, s), 1.46-1.56 (2H, m), 1.56-1.61 (1H, m), 2.03 (1H, dq, J=1.5, 7.3Hz), 4.45 (1H, dq, J=6.6, 1.5Hz), 5.32 (1H, m).
   ¹³C-NMR (C₆D₆) : d =14.01, 22.16, 23.47, 25.70, 37.33, 40.24, 74.63, 119.6, 139.9, 178.3.

### Comparative Example Production of Mixture (1:1(V/V)) of Naturally Occurring-Type Wine Lactone and an isomer thereof (3R, 3aR, 7aS)-3,6-dimethyl-3a,4,5,7a-tetrahydro-1-benzofuran-2(3H)-one

A mixture (1:1(v/v)) of naturally occurring-type wine lactone and an isomer thereof (3R, 3aR, 7aS)-3,6-dimethyl-3a,4,5,7a-tetrahydro-1-benzofuran-2(3H)-one can be obtained by subjecting Compound (2) prepared according to Step (A) of Example 1 to Steps (F) and (G) shown below in.

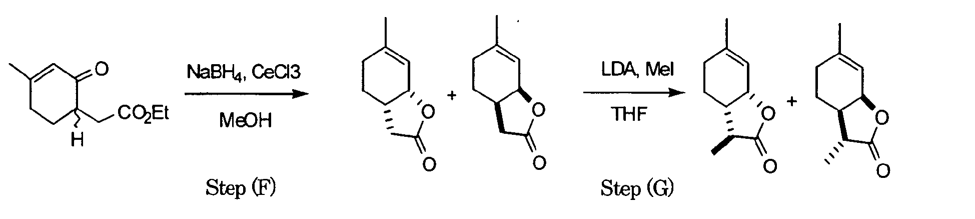

### (i) Step (F): Step of Obtaining Lactone by Reducing Carbonyl Group in Alky-adduct.

Step (F) is a reduction reaction wherein the 6-(carboethoxymethyl)-3-methyl-2-cyclohexene-1-one (Compound (2)) obtained by Step (A) of Example 1 was reduced to an alcohol. Specifically, Compound (2) was dissolved in methanol and cooled to 0°C, 1 to 2 mol of cerium chloride and 1 to 2 mol of sodium hydroborate were added per 1 mol of Compound (2), and then reacted at a temperature within the range from 0 to 10°C for about 5 to 30 minutes. By reducing the carbonyl group in the presence of cerium chloride, 1,4-reduction can be prevented. A lactone was formed between the hydroxyl group and ester which were simultaneously formed when the carbonyl group was reduced. In this case, the lactone obtained was an equal-ratio mixture of (3aS,7aR)-tetrahydro-6-methyl-2(3H)-benzofuranone and (3aR,7aS)-tetrahydro-6-methyl-2(3H)-benzofuranone.

### (ii) Step (G): Step of Obtaining a Mixture of Wine Lactone and Stereoisomer from Lactone

Step (G) can be conducted by methylating the equal-ratio lactone mixture obtained in Step (F) using methyl halide such as methyl bromide, methyl iodide, etc. Specifically, per 1 mol of the equal-ratio lactone mixture prepared in Step (F) [i.e., an equal-ratio mixture of (3aS,7aR)-tetrahydro-6-methyl-2(3H)-benzofuranone and (3aR,7aS)-tetrahydro-6-methyl-2(3H)-benzofuranone] were added 1 to 2 mol of lithium diisopropylamide (LDA) and 1 to 2 mol of methyl halide, and reacted at a temperature range from -78°C to +30°C for about 0.5 to 1 hour. An equal-ratio mixture of wine lactone [(3S,3aS,7aR)-3,6-dimethyl-3a,4,5,7a-tetrahydro-1-benzofuran-2(3H)-one] and isomer of wine lactone [(3R,3aR,7aS)-tetrahydro-3,6-dimethyl-3a,4,5,7a-tetrahydro-1-benzofuran-2(3H)-one] was thereby obtained.

### Example 2: Grapefruit Flavor

Grapefruit flavors (Flavors A and B) comprising the following components were prepared by employing a known method. Note that Flavor A contains an ethanol solution of naturally occurring-type wine lactone (Compound (6)) prepared in Example 1.

### <Components>

| (parts by weight) | | |
|---|---|---|
| | Flavor A | Flavor B |
| Grapefruit oil | 5.5 | 5.5 |
| Grapefruit aroma | 5.0 | 5.0 |
| Octanal | 0.3 | 0.3 |
| Decanal | 0.2 | 0.2 |
| Nootkatone | 0.1 | 0.1 |
| Ethyl alcohol | 55.0 | 55.0 |
| Glycerol | 5.0 | 5.0 |
| Water | 27.9 | 28.9 |
| 0.1 ppb wine lactone ethanol solution | 1.0 | --- |
| Total | 100.0 | 100.0 |

Flavor A obtained by adding wine lactone to the grapefruit oil and aroma attains more fresh fruit texture and has stronger natural grapefruit flavor than Flavor B without wine lactone.

### Example 3: Grapefruit Juice

Grapefruit juices (Beverages A and B) comprising the following components were prepared by employing a known method.

### <Components>

| (parts by weight) | | |
|---|---|---|
| | Beverage A | Beverage B |
| High fructose corn syrup | 5.0 | 5.0 |
| Citric acid, anhydrous | 0.15 | 0.15 |
| L-ascorbic acid | 0.02 | 0.02 |
| Grapefruit Flavor A in Example 2 | 0.1 | --- |
| Grapefruit Flavor B in Example 2 | --- | 0.1 |
| Water | 94.73 | 94.73 |
| Total | 100.0 | 100.0 |

Beverage A prepared using Flavor A with wine lactone had enhanced heavy-texture and juicy-taste of natural grapefruit, more than Beverage B prepared using Flavor B without wine lactone.

### Example 4: Comparison between Naturally Occurring-Type Wine Lactone alone and a Mixture of Naturally Occurring-Type Wine Lactone and Stereoisomer

Grapefruit juices (Beverages C and D) comprising the following components were prepared by employing a known method. As shown below, Beverage C comprises a solution obtained by dissolving naturally occurring-type wine lactone (Compound (6)) prepared in Example 1 into ethanol (0.001 ppb wine lactone ethanol solution). Beverage D comprises a solution obtained by dissolving an equal-ratio mixture of wine lactone[(3S,3aS,7aR)-3,6-dimethyl-3a,4,5,7a-tetrahydro-1-benzofuran-2(3H)-one] and its isomer [(3R,3aR,7aS)-3,6-dimethyl-3a,4,5,7a-tetrahydro-1-benzofuran-2 (3H)- one] in ethanol (0.001 ppb), prepared in Comparative Example.

### <Components>

| (parts by weight) | | |
|---|---|---|
| | Beverage C | Beverage D |
| High fructose corn syrup | 5.0 | 5.0 |
| Citric acid, anhydrous | 0.15 | 0.15 |
| L-ascorbic acid | 0.02 | 0.02 |
| Grapefruit Flavor B of Example 2 | 0.1 | 0.1 |
| Water | 94.63 | 94.63 |
| 0.001 ppb naturally occurring-type wine lactone ethanol solution | 0.1 | --- |
| Ethanol solution comprising 0.001 ppb naturally occurring-type wine lactone: isomer thereof = 1:1 | --- | 0.1 |
| Total | 100.0 | 100.0 |

Beverage C to which naturally occurring-type wine lactone alone was added had satisfactory sweetness and juicy-taste (i.e., fresh fruit texture). In contrast, Beverage D containing an equal-ratio mixture of naturally occurring-type wine lactone and isomers thereof had less juicy-taste than Beverage C, and an unpleasant sweetness.

### INDUSTRIAL APPLICABILITY

The present invention provides flavoring and perfume compositions containing wine lactone for use in foods and beverages, oral compositions and like orally taken or used products; soaps, perfumes, fragrances, shampoos and like cosmetics and toiletries. The flavoring and perfume compositions of the present invention can improve the heavy-texture, and, in the case of fruit-based flavors, improve the juicy-taste of a product. By using the flavoring and perfume compositions of the present invention, the flavor of the original material can be reproduced well, and can improve the reality of the flavor and fragrance.

## Claims

1. A method for producing (1R,6S)-6-(carboalkoxymethyl)-3-methyl-2-cyclohexene-1-ol (Compound (3)) represented by the formula below: 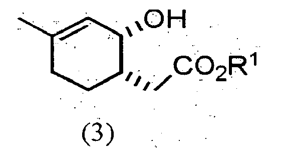 wherein R¹ is a C₁₋₄ lower alkyl group;
the method comprising the step of reducing the carbonyl group in 6-(carboalkoxymethyl)-3-methyl-2-cyclohexene1-one (Compound (2)) represented by the formula below: 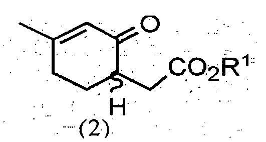 wherein R¹ is the same as the above;
by using an optically active oxazaborolidine as an asymmetric catalyst.

2. The method for producing (1R,6S)-6-(carboalkoxymethyl)-3-methyl-2-cyclohexene-1-ol (Compound (3)) according to claim 1, wherein the optically active oxazaborolidine is (R)-5,5-diphenyl-2-methyl-3,4-propano-1,3,2-oxazaborolidine.

3. A method for producing (3S,3aS,7aR)-3,6-dimethyl-3a,4,5,7a-tetrahydro-1-benzofuran-2(3H)-one (Compound (6)) comprising steps (A)-(E) below:
(1) Step (A): subjecting 3-methyl-2-cyclohexene-1-one (Compound (1)) to an alkyl addition reaction to obtain 6-(carboalkoxymethyl)-3-methyl-2-cyclohexene-1-one (Compound (2));
(2) Step (B): reducing the carbonyl group in the obtained 6-(carboalkoxymethyl) -3-methyl-2-cyclohexene-1-one (Compound (2)) using an optically active oxazaborolidine as an asymmetric catalyst to obtain (1R,6S)-6-(carboalkoxymethyl)-3-methyl-2-cyclohexene-1-ol (Compound (3));
(3) Step (C): hydrolyzing the ester moiety of the obtained (1R,6S)-6-(carboalkoxymethyl)-3-methyl-2-cyclohexene-1-ol (Compound (3)) to obtain (1R,6S)-6-carboxymethyl -3-methyl-2-cyclohexene-1-ol (Compound (4));
(4) Step (D): lactonizing the obtained (1R,6S)-6-carboxymethyl-3-methyl-2-cyclohexene-1-ol (Compound (4)) to obtain (3aS,7aR)-tetrahydro-6-methyl-2(3H)-benzofuranone (Compound (5)); and
(5) Step (E): methylating the obtained (3aS,7aR)-tetrahydro-6-methyl-2(3H)-benzofuranone (Compound (5)) to obtain (3S,3aS,7aR)-3,6-dimethyl-3a,4,5,7a-tetrahydro-l-benzofuran-2(3H)-one (Compound (6)). 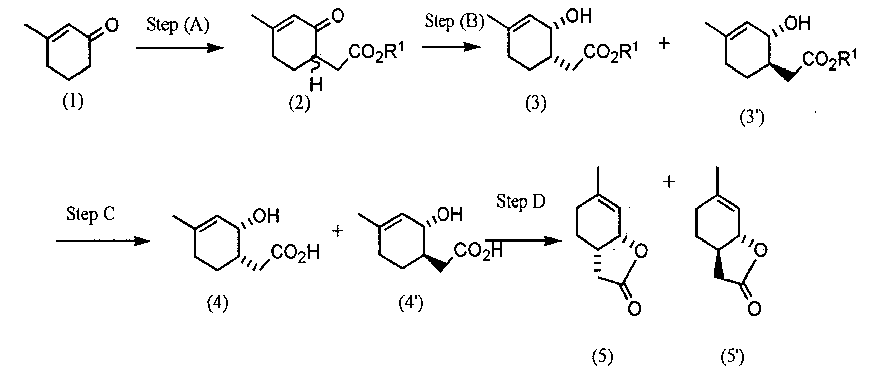 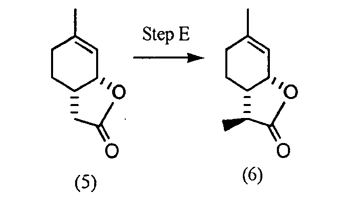
wherein R¹ is a C₁₋₄ lower alkyl group.

4. The method for producing (3S,3aS,7aR)-3,6-dimethyl-3a,4,5,7a-tetrahydro-1-benzofuran-2(3H)-one (Compound (6)) according to claim 3, wherein the optically active oxazaborolidine used in Step (B) is (R)-5,5-diphenyl-2-methyl-3,4-propano-1,3,2-oxazaborolidine.

5. A (3S,3aS,7aR)-3,6-dimethyl-3a,4,5,7a-tetrahydro-1-benzofuran-2(3H)-one obtained by the method of claim 4.

6. A flavoring or perfume composition containing a flavoring or perfume component and 10⁻⁷ to 10⁴ ppb of (3S,3aS,7aR)-3,6-dimethyl-3a,4,5,7a-tetrahydro-1-benzofuran-2(3H)-one.

7. The flavoring or perfume composition according to claim 6, wherein the flavoring or perfume component is a fruit-based flavoring or perfume component.

8. A method for improving the texture of a flavoring or perfume composition comprising the step of adding (3S,3aS,7aR)-3,6-dimethyl-3a,4,5,7a-tetrahydro-1-benzofuran-2(3H)-one to the flavoring or perfume composition in addition to a flavoring or perfume component in such a manner that the concentration of the ((3S,3aS,7aR)-3,6-dimethyl-3a,4,5,7a-tetrahydro-1-benzofuran-2(3H)-one is 10⁻⁷ to 10⁴ ppb.

9. The method according to claim 8, wherein the texture is a flavor or fragrance, heavy-texture, or juicy-taste.

10. Foods and beverages containing 10⁻¹⁰ to 10² ppb of (3S,3aS,7aR)-3,6-dimethyl-3a,4,5,7a-tetrahydro-1-benzofuran-2(3H)-one.

11. A method for improving the flavor of foods and beverages comprising the step of adding 10⁻¹⁰ to 10² ppb of (3S,3aS,7aR)-3,6-dimethyl-3a,4,5,7a-tetrahydro-1-benzofuran-2(3H)-one to the foods and beverages.

12. The method for improving the flavor of foods and beverages according to claim 11, wherein the flavor is a juicy-taste and heavy-texture.
